# FASCICULE DE BREVET EUROPEEN

(11) **EP 2 365 836 B1**
(45) Date de publication et mention de la délivrance du brevet: **30.05.2018**
(21) Numéro de dépôt: 09795455.6
(22) Date de dépôt: 18.11.2009
(51) Int. Cl.: A61M 25/10, A61M 25/00, A61M 29/02, A61B 17/22

(54) **DISPOSITIF D'ANGIOPLASTIE**
ANGIOPLASTIE-VORRICHTUNG
ANGIOPLASTY DEVICE

(30) Priorité: 19.11.2008 FR 0806472
(43) Date de publication de la demande: 21.09.2011
(60) Demande divisionnaire: 18167034.0
(73) Titulaire: NEXSTEP MEDICAL, 21000 Dijon (FR)
(72) Inventeur: Sarradon, Pierre, 83270 Saint-Cyr-Sur-Mer (FR)
(74) Mandataire: Alatis
(86) Numéro de dépôt international: PCT/FR2009/001326
(87) Numéro de publication internationale: WO 2010/058103

(56) Documents cités:
- WO-A-2007/132447
- US-A- 4 748 984
- US-A- 4 892 519
- US-A- 4 909 258
- US-A- 5 344 402
- US-A1- 2009 036 831
- US-B1- 6 322 577

## Description

### DOMAINE TECHNIQUE DE L'INVENTION

L'invention se rapporte à un dispositif d'angioplastie destiné à être introduit dans un conduit du corps humain, qui est de préférence un vaisseau sanguin, afin de permettre l'élargissement local du conduit. Ce dispositif est particulièrement utile pour traiter les occlusions ou les sténoses des vaisseaux sanguins.

### ÉTAT DE LA TECHNIQUE ANTÉRIEURE

Pour traiter une sténose ou une occlusion d'un vaisseau sanguin, on peut utiliser un dispositif d'angioplastie qui se compose d'une tige sur laquelle est fixé un ballonnet gonflable. Un tel dispositif est par exemple décrit dans le document EP1897584. Ce dispositif comporte une tige pourvue d'un ballonnet gonflable dans laquelle sont percés deux conduits. Le premier conduit est agencé pour recevoir un guide souple filaire permettant de guider le cheminement du dispositif dans le vaisseau jusqu'à la zone à traiter, tandis que le deuxième conduit est en communication fluidique avec le ballonnet permettant ainsi l'injection d'un fluide sous pression dans le ballonnet, en l'occurrence du sérum physiologique, le cas échéant mélangé à un produit de contraste iodé.

Pour mettre en place ce dispositif, on peut injecter un produit de contraste dans le vaisseau sanguin afin de localiser la sténose. Un guide filaire est ensuite introduit dans le vaisseau à traiter afin de faciliter l'insertion du dispositif d'angioplastie au niveau de la sténose. Un guide filaire, ainsi que son procédé d'utilisation, sont par exemple décrits dans le document FR2533130.

Le dispositif d'angioplastie est ensuite introduit autour du guide filaire de façon à ce que le guide filaire se trouve dans le premier conduit. Une fois que le ballonnet est introduit au niveau de la sténose, un fluide sous pression est injecté dans le ballonnet à l'aide du deuxième conduit. Le gonflement du ballonnet permet d'élargir le vaisseau sanguin au niveau de la sténose. Il est ensuite nécessaire de visualiser à nouveau le vaisseau sanguin pour vérifier que la sténose a bien été supprimée. Pour cela, le cathéter à ballonnet est retiré et remplacé par un cathéter d'angiographie, à extrémité multiperforée, qui est positionné en regard ou en amont de la lésion traitée. Le guide est ensuite retiré et un produit de contraste iodé est injecté dans la lumière du cathéter d'angiographie. Si une nouvelle angioplastie est requise, le guide peut être à nouveau monté sur le cathéter d'angiographie, le cathéter peut être ensuite retiré et remplacé par le cathéter à ballonnet.

Alternativement, par soucis de simplification, le guide filaire peut être retiré et l'injection du produit de contraste peut se faire par la lumière libérée par le guide filaire, pour autant que le diamètre de cette lumière de guidage soit suffisant, sachant que le produit de contraste est visqueux. Une telle solution est illustrée par exemple par le document WO 2007 132 447. Dans le cas où la sténose n'a pas totalement été supprimée, le ballonnet doit être repositionné à l'endroit où le vaisseau sanguin est rétréci. Pour cela, un guide filaire doit être réintroduit dans le premier conduit pour permettre le repositionnement du ballonnet au niveau du rétrécissement du vaisseau sanguin. Cette opération de repositionnement du ballonnet peut être réitérée plusieurs fois tant que la sténose n'a pas complètement été supprimée.

Le traitement d'une sténose, ou d'une occlusion, comporte donc une succession de phases de traitement, pendant lesquelles le ballonnet est gonflé, et de phases de visualisation, pendant lesquelles un produit de contraste est injecté. Entre chaque phase de traitement et de visualisation, le praticien doit, pour pratiquer l'injection, soit retirer le ballonnet et le remplacer par un cathéter d'angiographie, soit retirer le guide filaire pour utiliser le canal libéré. Ces introductions et retraits successifs du guide filaire dans le premier conduit sont longues et fastidieuses pour le praticien.

Un autre inconvénient de ces techniques vient du fait que la répartition du produit de contraste dans le vaisseau sanguin se fait toujours dans le sens de circulation du sang, de sorte que si le vaisseau comporte des ramifications entre le point d'injection et le point de la lésion, une partie du produit de contraste est diffusée inutilement dans les vaisseaux adjacents.

En outre, dans les dispositifs de l'art antérieur, le produit de contraste est injecté dans le vaisseau sanguin à l'aide du premier conduit, si bien qu'il est injecté dans la totalité du vaisseau sanguin. De grandes quantités de produit de contraste sont donc injectées dans le sang du patient, ce qui peut être néfaste pour sa santé.

On connaît par ailleurs un dispositif d'angioplastie composé d'un cathéter pourvu d'un ballonnet équipé d'orifices d'injection situés au niveau du ballonnet. Ces orifices, reliés à l'extrémité proximale du cathéter par un conduit, permettent, après gonflage du ballonnet au niveau de l'occlusion, d'injecter contre la paroi de l'occlusion un médicament destiné au traitement de la paroi du vaisseau. Un tel dispositif est décrit dans le document US 5 344 402. Pour permettre la circulation du sang dans le vaisseau durant le traitement, un conduit traversant le ballonnet relie des orifices situés en amont et en aval du ballonnet.

Dans le document US 4 909 258 est illustré un dispositif d'angioplastie comportant un corps tubulaire à trois conduits, à savoir un premier pour le passage d'un guide filaire, un deuxième pour le gonflage d'un ballon d'occlusion, et un troisième qui peut servir soit à l'injection d'un produit de contraste, soit à l'insertion d'un cathéter pourvu d'un ballon de dilatation ayant vocation à traiter une sténose, alors que le ballon d'occlusion reste gonflé pour stabiliser le positionnement du dispositif. Mais ce dispositif ne permet pas d'acheminer de liquide de contraste directement à proximité du ballon de dilatation.

### EXPOSÉ DE L'INVENTION

L'invention vise à remédier aux inconvénients de l'état de la technique en proposant un dispositif d'angioplastie transluminale percutanée permettant de visualiser le vaisseau sanguin sans effectuer de manoeuvre supplémentaire. L'invention vise aussi à améliorer la qualité de la visualisation, tout en diminuant les quantités de produit de contraste injectées dans le sang du patient.

Pour ce faire, l'invention se définit par un dispositif d'angioplastie selon la revendication 1.

Le dispositif d'angioplastie comporte donc un troisième conduit et au moins un orifice d'injection situé dans la partie distale du corps tubulaire. Cet orifice d'injection permet d'injecter un produit de contraste dans le sang sans avoir à retirer le guide filaire. Le dispositif d'angioplastie peut donc être repositionné très facilement et très rapidement. L'orifice d'injection se trouve préférentiellement à moins de 5 cm du ballonnet. Dans le cas où le corps tubulaire comporte plusieurs orifices d'injection, l'orifice d'injection le plus éloigné du ballonnet, se trouve préférentiellement à moins de 5 cm du ballonnet.

Ce dispositif peut donc être utilisé pour le diagnostic des sténoses ou des occlusions, et il peut également être utilisé pour leur traitement. Ce dispositif permet également de vérifier que le gonflage du ballonnet a bien permis de supprimer l'occlusion ou la sténose sans avoir à enlever le guide filaire.

Ce dispositif joue donc tout à la fois le rôle de sonde de visualisation et le rôle d'instrument pour le traitement des sténoses ou des occlusions.

Par ailleurs, le produit de contraste est injecté localement grâce à l'orifice d'injection situé à proximité du ballonnet, ce qui permet de visualiser très précisément la zone d'intérêt, tout en injectant une petite quantité de produit de contraste dans le sang du patient.

La qualité de la visualisation du produit de contraste peut également être améliorée en répartissant des orifices d'injection radialement autour du corps tubulaire, en aval et en amont du ballonnet, ce qui permet d'avoir une répartition satisfaisante du produit de contraste, et ce quel que soit le sens de circulation du sang.

Le deuxième conduit est étanche pour éviter que le fluide qui permet de gonfler le ballonnet ne passe dans le sang du patient.

Avantageusement, selon un mode de revendication le premier conduit est circulaire et il a un diamètre sensiblement égal à celui du guide filaire. De cette façon, le conduit et le guide filaire sont bien en contact et donc le guidage du dispositif d'angioplastie par le guide filaire est facilité.

La section du troisième conduit doit être choisie de manière à satisfaire à deux contraintes contradictoires : elle doit en effet d'une part être la plus grande possible pour permettre l'injection à haut débit du produit de contraste dont la viscosité est élevée, et d'autre part d'une dimension minimale pour faciliter conserver un diamètre global du dispositif le plus petit possible.

Suivants différents modes de réalisation :
- le corps tubulaire peut comporter une pluralité d'orifices d'injection, en communication fluidique avec le troisième conduit, les orifices d'injection étant répartis radialement autour du corps tubulaire. Cette répartition des orifices d'injection permet une meilleure répartition du milieu de visualisation dans la zone à traiter ;
- le ou les orifices d'injection incluent au moins un orifice d'injection aval situé en aval du ballonnet;
- le ou les orifices d'injection incluent au moins un orifice d'injection amont situé en amont du ballonnet. La répartition des orifices d'injection en amont et en aval du ballonnet permet d'améliorer la visualisation de la sténose, quel que soit le sens de circulation du sang ;
- les premier, deuxième et troisième conduits peuvent être concentriques. Dans ce cas, le premier conduit est au centre afin d'avoir un bon maintien du guide filaire, le deuxième conduit entoure le premier conduit et le troisième conduit entoure le premier et le deuxième conduits pour permettre une meilleure visualisation de la zone dans laquelle se trouve le ballonnet ; cette disposition peut permettre un gain de place, de sorte que le diamètre du corps tubulaire dans son ensemble se trouve réduit et ainsi l'insertion du dispositif d'angioplastie dans le sang est facilitée ;
- les premier, deuxième et troisième conduits sont disposés côte à côte, ce qui facilite la fabrication du dispositif ;
- l'extrémité proximale du corps tubulaire est pourvue de trois embouts, chaque embout étant relié de manière étanche à un des conduits ;
- le premier conduit débouche d'une part à l'extrémité proximale et d'autre part à l'extrémité distale, ce qui permet le passage du guide filaire d'une extrémité à l'autre ;
- le premier conduit débouche d'une part à une position intermédiaire entre l'extrémité proximale et l'extrémité distale et d'autre part à l'extrémité distale, ce qui permet de faciliter les manipulations entre le corps tubulaire et le guide filaire.
L'invention concerne également un corps tubulaire selon un des modes de réalisation décrit précédemment.

### BRÈVE DESCRIPTION DES FIGURES

D'autres caractéristiques et avantages de l'invention ressortiront à la lecture de la description qui suit, en référence aux figures annexées, qui illustrent :
- la figure 1, une vue schématique d'un dispositif d'angioplastie selon un premier mode de réalisation de l'invention;
- la figure 2, une vue en coupe transversale du corps tubulaire du dispositif d'angioplastie de la figure 1 ;
- la figure 3, une vue en coupe transversale du corps tubulaire d'un dispositif d'angioplastie selon un deuxième mode de réalisation de l'invention;
- la figure 4, une vue en coupe transversale du corps tubulaire d'un dispositif d'angioplastie selon un troisième mode de réalisation de l'invention ;
- la figure 5, une vue schématique d'un dispositif d'angioplastie selon un quatrième mode de réalisation de l'invention.

Pour plus de clarté, les éléments identiques ou similaires sont repérés par des signes de référence identiques sur l'ensemble des figures.

### DESCRIPTION DETAILLEE D'UN MODE DE RÉALISATION

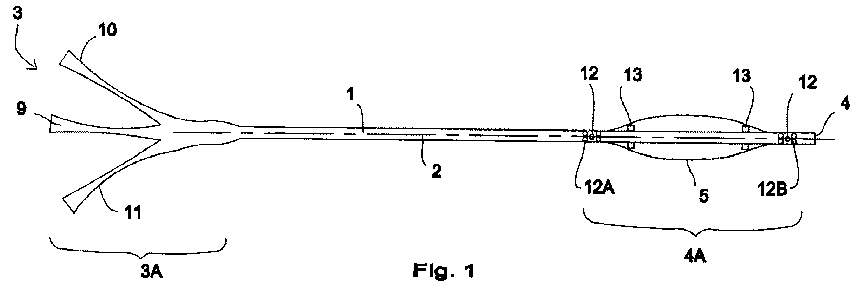

Le dispositif d'angioplastie représenté sur la figure 1 comprend un corps tubulaire 1 réalisé dans un matériau flexible et biocompatible, notamment un matériau synthétique qui s'étend suivant un axe longitudinal 2 entre une extrémité proximale 3 et une extrémité distale 4. Le corps tubulaire comprend en outre une partie proximale 3A incluant l'extrémité proximale 3 et une partie distale 4A incluant l'extrémité distale 4. Le corps tubulaire 1 est pourvu dans sa partie distale 4A d'un ballonnet 5 gonflable qui entoure radialement le corps tubulaire.

Le corps tubulaire 1 comporte en outre des orifices d'injection 12 percés dans la paroi du corps tubulaire et qui sont disposés radialement en amont (12B) et en aval (12A) du ballonnet gonflable (5). Ces orifices d'injection 12 sont disposés à proximité du ballonnet gonflable. Les termes amont et aval renvoient au sens d'écoulement du fluide dans le dispositif d'angioplastie.

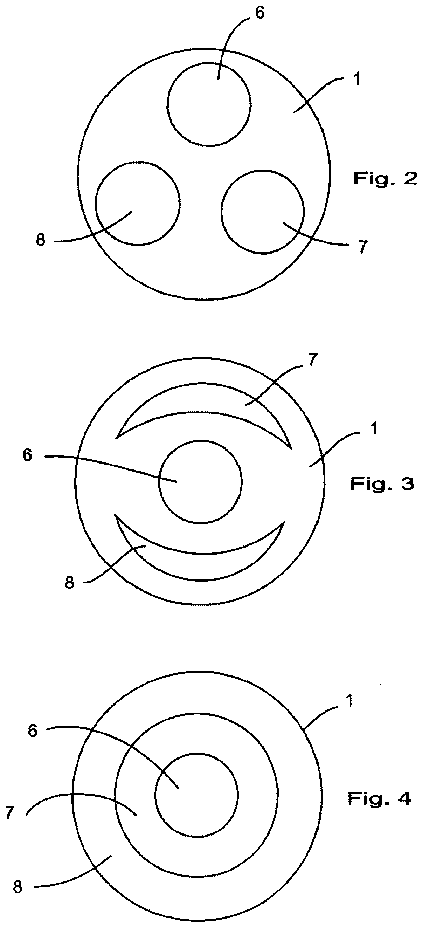

Le corps tubulaire est traversé par trois conduits 6, 7, 8, représentés en coupe sur la figure 2. Les trois conduits 6, 7 et 8 s'étendent parallèlement à l'axe longitudinal. Le premier conduit 6 s'étend sur toute la longueur du corps tubulaire de sorte qu'il relie les extrémités proximale 3 et distale 4 du corps tubulaire. Le premier conduit 6 est de section circulaire et il présente un diamètre intérieur sensiblement égal au diamètre extérieur du guide filaire qui permettra la mise en place du dispositif d'angioplastie dans la veine. Ainsi, le dispositif d'angioplastie est mieux guidé par le guide filaire. L'extrémité proximale 3 est en outre pourvue d'un embout 9 qui est relié au premier conduit 6. L'introduction du guide filaire dans le premier conduit 6 a lieu par l'embout 9.

Le deuxième conduit 7 relie de manière étanche l'extrémité proximale 3 du corps tubulaire au ballonnet gonflable 5. L'extrémité proximale 3 est pourvue d'un deuxième embout 10 qui est relié de manière étanche au deuxième conduit. L'embout 10 est destiné à être relié à un dispositif permettant d'injecter un fluide sous pression dans le deuxième conduit 7 afin de gonfler le ballonnet. Avantageusement, ce fluide sous pression est un mélange de sérum physiologique et de produit de contraste.

Le troisième conduit 8 relie l'extrémité proximale 3 du corps tubulaire aux orifices d'injection 12. L'extrémité proximale 3 est en outre pourvue d'un troisième embout 11 qui est connecté de manière étanche au troisième conduit 8. Le troisième conduit 8 permet l'injection d'un produit de contraste dans le vaisseau sanguin afin de visualiser le rétrécissement du vaisseau sanguin à traiter et le contrôler après l'angioplastie.

En outre, afin de faciliter le positionnement du ballonnet à l'endroit du rétrécissement, en particulier lorsque le ballonnet n'est pas gonflé, une bague de positionnement 13 est placée dans le ballonnet. Cette bague de positionnement 13 est repérable, tout comme le produit de contraste, au moyen de rayons X. Dans un mode de réalisation préféré, le corps tubulaire peut comprendre deux bagues de positionnement, chacune de ces bagues étant placées à la limite du ballonnet afin de le repérer.

L'agencement des conduits à l'intérieur du corps tubulaire peut varier comme on peut le voir sur les figures 3 et 4. Dans les figures 3 et 4, le premier conduit 6 présente une section circulaire de diamètre sensiblement égal à celui du guide filaire qui sera introduit dans ce premier conduit 6. Dans l'agencement de la figure 3, les deuxième et troisième conduits 7 et 8 sont disposés en demi-lune autour du premier conduit, tandis que dans l'agencement de la figure 4, les trois conduits 6, 7 et 8 sont de section transversale circulaire et ils sont concentriques. Le premier conduit 6 est au centre du corps tubulaire, et il est entouré par le deuxième conduit 7, lui-même entouré par le troisième conduit 8.

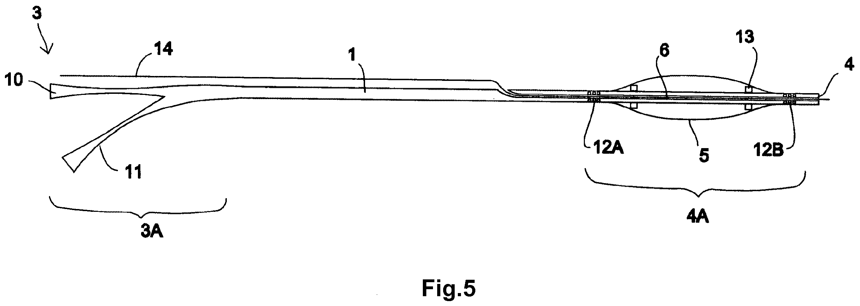

La figure 5 représente un dispositif d'angioplastie selon un autre mode de réalisation de l'invention. Dans ce mode de réalisation, le corps tubulaire 1 comprend deux conduits seulement dans sa partie proximale 3A, le deuxième et le troisième conduits et il comprend trois conduits dans sa partie distale 4A. Sur cette partie où le corps tubulaire comprend uniquement deux conduits, le guide filaire 14 est extérieur au corps tubulaire 1, tandis que dans la partie distale où le corps tubulaire comprend trois conduits, le guide filaire passe dans le premier conduit.

Le traitement d'une sténose à l'aide du dispositif d'angioplastie de la figure 1 va maintenant être précisé.

Dans un premier temps, le praticien insère un cathéter dans la veine à traiter et il injecte un produit de contraste, comme par exemple de l'iode, dans le vaisseau sanguin à traiter. Grâce à ce produit de contraste, le praticien peut observer le vaisseau sanguin en radiographie et il peut ainsi localiser la sténose à traiter. Le praticien introduit ensuite le guide filaire dans le vaisseau à traiter, puis il insert le dispositif d'angioplastie dans le vaisseau sanguin en faisant glisser le premier conduit sur le guide filaire. Le guide filaire facilite l'introduction du dispositif d'angioplastie malgré la souplesse et la longueur de ce dernier.

Le praticien place le ballonnet au niveau du rétrécissement grâce à la bague de marquage 13 qui est radiomarquée. Une fois que le ballonnet a atteint le segment rétréci du vaisseau sanguin, le praticien gonfle le ballonnet à l'aide du deuxième conduit 7. Les dépôts athéromateux qui rétrécissaient le vaisseau sont alors comprimés contre les parois du vaisseau sanguin, ce qui permet d'augmenter le diamètre de sa lumière interne. Une fois cette opération effectuée, le ballonnet est dégonflé, toujours à l'aide du deuxième conduit 7.

Le praticien injecte ensuite un produit de contraste, à l'aide du troisième conduit, dans le sang du patient sans avoir à effectuer les manipulations nécessaires dans l'art antérieur. L'injection de ce produit de contraste permet au praticien de visualiser le vaisseau sanguin après le gonflement du ballonnet de façon à pouvoir vérifier que le segment rétréci a bien été supprimé. Dans le cas où ce segment rétréci n'a pas été totalement supprimé par le premier gonflement, le praticien peut déplacer le ballonnet pour le repositionner au niveau de la portion rétrécie restante. La détection de cette portion rétrécie restante se fait à l'aide du produit de contraste. Une fois le ballonnet repositionné, le praticien peut regonfler le ballonnet. Il peut ensuite à nouveau vérifier que cette opération s'est bien passée. Le praticien peut réitérer ces opérations d'élargissement/vérification autant de fois que nécessaire, et ce, sans avoir à effectuer de manipulation supplémentaire, ce qui lui permet un gain de temps important, et évite certains incidents possibles lors de la manipulation. En outre, le produit de contraste est injecté localement, ce qui permet de très bien visualiser la zone d'intérêt, sans trop injecter de produit de contraste dans le sang du patient.

Naturellement l'invention n'est pas limitée aux exemples de réalisation ci-dessus, et diverses modifications ou variantes peuvent être envisagées. Par exemple, d'autres agencements des trois conduits peuvent être envisagés. On peut également envisager d'ajouter un quatrième conduit dans le corps tubulaire, par exemple pour injecter des médicaments ou des agents actifs dans le sang.

Par ailleurs, le corps tubulaire peut soit être un cylindre plein qui est percé par trois conduits, ou alors il peut se présenter sous la forme d'une paroi externe cylindrique qui entoure les trois conduits..

## Revendications

1. Dispositif d'angioplastie comportant :
- un guide filaire destiné à être inséré dans un vaisseau sanguin,
- un corps tubulaire (1) flexible s'étendant suivant un axe longitudinal (2) entre une extrémité proximale (3) et une extrémité distale (4) et présentant une partie proximale (3A) incluant l'extrémité proximale (3) et une partie distale (4A) incluant l'extrémité distale (4), le corps tubulaire (1) comportant:
- un ballonnet gonflable (5) disposé radialement autour du corps tubulaire (1) dans la partie distale (4A) et apte à être gonflé pour augmenter le diamètre interne du vaisseau sanguin;
- un premier conduit (6) interne étanche s'étendant parallèlement à l'axe longitudinal (2) au moins dans la partie distale (4A) et débouchant d'une part à l'extrémité proximale ou à une position intermédiaire entre l'extrémité proximale et le ballonnet gonflable (5) et d'autre part à l'extrémité distale (4) de façon à permettre l'insertion du guide filaire dans le premier conduit et le coulissement du corps tubulaire le long du guide filaire ;
- au moins un deuxième conduit (7) interne étanche s'étendant parallèlement à l'axe longitudinal (2) débouchant d'une part à l'extrémité proximale (3) et d'autre part dans le ballonnet (5) de façon à relier de manière étanche l'extrémité proximale (3) du corps tubulaire au ballonnet gonflable (5) et à permettre l'injection d'un fluide dans le ballonnet;
- un ou plusieurs orifices d'injection (12) situés dans la partie distale (4A) et incluant au moins un orifice d'injection aval (12B) situé en aval du ballonnet (5) et/ou au moins un orifice d'injection amont (12B) situé en amont du ballonnet (5) ; et
- un troisième conduit (8) interne étanche débouchant d'une part à l'extrémité proximale (3) et d'autre part dans le ou les orifices d'injection (12) de façon à permettre l'injection d'un produit de contraste à travers le ou les orifices d'injection (12) lorsque le guide filaire est dans le premier conduit et que le ballonnet gonflable (5) est dégonflé, le troisième conduit (8) n'étant pas ouvert sur le premier conduit (6).

2. Dispositif d'angioplastie selon la revendication 1, **caractérisé en ce que** les orifices d'injection (12) sont répartis radialement autour du corps tubulaire (1).

3. Dispositif d'angioplastie selon l'une quelconque des revendications précédentes, **caractérisé en ce que** le ou les orifices d'injection (12) incluent au moins un orifice d'injection aval (12B) situé en aval du ballonnet (5).

4. Dispositif d'angioplastie selon l'une quelconque des revendications précédentes, **caractérisé en ce que** le ou les orifices d'injection (12) incluent au moins un orifice d'injection amont (12B) situé en amont du ballonnet (5).

5. Dispositif d'angioplastie selon l'une quelconque des revendications précédentes, **caractérisé en ce que** les premier, deuxième et troisième conduits (6, 7, 8) sont concentriques.

6. Dispositif d'angioplastie selon l'une quelconque des revendications précédentes, **caractérisé en ce que** les premier, deuxième et troisième conduits (6, 7, 8) sont disposés côte à côte.

7. Dispositif d'angioplastie selon l'une quelconque des revendications précédentes, **caractérisé en ce que** le premier conduit (6) est de section circulaire et de diamètre égal à celui du guide filaire.

8. Dispositif d'angioplastie selon l'une quelconque des revendications précédentes, **caractérisé en ce que** l'extrémité proximale (3) du dispositif est pourvue de trois embouts (9, 10, 11), chaque embout (9, 10, 11) étant relié de manière étanche à un des conduits (6, 7, 8).

9. Dispositif d'angioplastie selon l'une quelconque des revendications précédentes, **caractérisé en ce que** le corps tubulaire (1) comporte un quatrième conduit, par exemple pour injecter des médicaments ou des agents actifs dans le sang.

10. Dispositif d'angioplastie selon l'une quelconque des revendications précédentes, **caractérisé en ce que** le premier conduit débouche d'une part à l'extrémité proximale et d'autre à l'extrémité distale.

11. Dispositif d'angioplastie selon l'une quelconque des revendications 1 à 10, **caractérisé en ce que** le premier conduit débouche à une position intermédiaire entre l'extrémité proximale et le ballonnet gonflable (5) et d'autre part à l'extrémité distale.

## Patentansprüche

1. Angioplastische Vorrichtung bestehend aus:
- einer Drahtführung, die dazu bestimmt ist, in ein Blutgefäß eingeführt zu werden,
- einem röhrenförmigen elastischen Körper (1), der sich auf einer Längsachse (2) zwischen einem proximalen Ende (3) und einem distalen Ende (4) erstreckt, und einen proximalen Teil (3A) mit dem proximalen Ende (3) und einen distalen Teil (4A) mit dem distalen Ende (4) aufweist, wobei der röhrenförmige Körper (1) folgendes umfasst:
- einen aufblasbaren Ballon (5), der im distalen Teil (4A) radial um den röhrenförmigen Körper (1) angeordnet ist, und aufgeblasen werden kann, um den Innendurchmesser des Gefäßes zu erweitern;
- eine erste innere dichte Durchführung (6), die sich mindestens im distalen Teil (4A) parallel zur Längsachse (2) erstreckt und zum einen bis zum proximalen Ende oder bis zu einer Zwischenposition zwischen dem proximalen Ende und dem aufblasbaren Ballon (5), und zum andern bis zum distalen Ende (4) führt, sodass die Drahtführung in die erste Durchführung eingeführt und der röhrenförmige Körper an der Drahtführung entlang verschoben werden kann;
- mindestens eine zweite innere dichte Durchführung (7), die sich parallel zur Längsachse (2) erstreckt und zum einen bis zum proximalen Ende (3), und zum anderen in den Ballon (5) führt, sodass das proximale Ende (3) dicht mit dem röhrenförmigen Körper des aufblasbaren Ballons (5) verbunden wird und das Einspritzen eines Mediums in den Ballon möglich ist;
- eine oder mehrere Einspritzöffnungen (12) im distalen Teil (4A), wobei mindestens eine Einspritzöffnung (12B) dem Ballon (5) vorgelagert und/oder mindestens eine Einspritzöffnung (12B) dem Ballon (5) nachgelagert ist, und
- eine dritte innere dichte Durchführung (8), die zum einen bis zum proximalen Ende (3) und zum andern in die Einspritzöffnung bzw. die Einspritzöffnungen (12) führt, so dass das Eispritzen eines Kontrastmittels durch die Einspritzöffnung oder Einspritzöffnungen (12) möglich ist, wenn sich die Drahtführung in der ersten Durchführung befindet und der aufblasbare Ballon (5) nicht aufgeblasen ist, wobei die dritte Durchführung (8) zur ersten Durchführung (6) hin nicht offen ist.

2. Angioplastische Vorrichtung nach Anspruch 1, **dadurch gekennzeichnet, dass** die Einspritzöffnungen (12) radial um den röhrenförmigen Körper (1) herum verteilt sind.

3. Angioplastische Vorrichtung nach einem der vorhergehenden Ansprüche, **dadurch gekennzeichnet, dass** mindestens eine Einspritzöffnung (12) eine hinter dem Ballon (5) nachgelagerte Einspritzöffnung (12B) ist.

4. Angioplastische Vorrichtung nach einem der vorhergehenden Ansprüche, **dadurch gekennzeichnet, dass** mindestens eine Einspritzöffnung (12) eine vor dem Ballon (5) vorgelagerte Einspritzöffnung (12B) ist.

5. Angioplastische Vorrichtung nach einem der vorhergehenden Ansprüche, **dadurch gekennzeichnet, dass** die erste, die zweite und die dritte Durchführung (6, 7, 8) konzentrisch sind.

6. Angioplastische Vorrichtung nach einem der vorhergehenden Ansprüche, **dadurch gekennzeichnet, dass** die erste, die zweite und die dritte Durchführung (6, 7, 8) nebeneinander angeordnet sind.

7. Angioplastische Vorrichtung nach einem der vorhergehenden Ansprüche, **dadurch gekennzeichnet, dass** die erste Durchführung (6) einen kreisförmigen Querschnitt aufweist und einen Durchmesser, der mit dem der Drahtführung identisch ist.

8. Angioplastische Vorrichtung nach einem der vorhergehenden Ansprüche, **dadurch gekennzeichnet, dass** das proximale Ende (3) der Vorrichtung mit drei Endstücken (9, 10, 11) versehen ist, wobei jedes Endstück (9, 10, 11) dicht mit einer der Durchführungen (6, 7, 8) verbunden ist.

9. Angioplastische Vorrichtung nach einem der vorhergehenden Ansprüche, **dadurch gekennzeichnet, dass** der röhrenförmige Körper (1) eine vierte Durchführung enthält, die beispielsweise zum Einspritzen von Arzneimitteln oder Wirkstoffen in das Blut verwendet werden kann.

10. Angioplastische Vorrichtung nach einem der vorhergehenden Ansprüche, **dadurch gekennzeichnet, dass** die erste Durchführung zum einen bis zum proximalen Ende, und zum anderen bis zum distalen Ende führt.

11. Angioplastische Vorrichtung nach einem der Ansprüche 1 bis 10, **dadurch gekennzeichnet, dass** die erste Durchführung bis zu einer Zwischenposition zwischen dem proximalen Ende und dem aufblasbaren Ballon (5), und zum anderen bis zum distalen Ende führt.

## Claims

1. An angioplasty device comprising :
- a guide wire to be inserted into a blood vessel,
- a flexible tubular body (1) extending along a longitudinal axis (2) between a proximal end (3) and a distal end (4) and having a proximal part (3A), which includes the proximal end (3), and a distal part (4A) which includes the distal end (4), with the tubular body (1) comprising :
- an inflatable balloon (5) arranged radially about the tubular body (1) in the distal part (4) and capable of being inflated to increase the internal diameter of the blood vessel;
- a first leaktight internal conduit (6) extending parallel to the longitudinal axis (2) at least in the distal part (4A) and opening out, on the one hand, at the proximal end or at an intermediate position between the proximal end and the inflatable balloon (5), and on the other hand at the distal end (4) in such a way as to permit the insertion of the guide wire into the first conduit and the sliding of the tubular body along the guide wire;
- at least one second leaktight internal conduit (7) extending parallel to the longitudinal axis (2) and opening out, on the one hand, at the proximal end (3), and on the other hand into the balloon (5), in such a way as to connect the proximal end (3) of the tubular body in a leaktight manner with the inflatable balloon (5) and to permit the injection of a fluid into the balloon;
- one or more injection orifice (s) (12) situated in the distal part (4A) and including at least one downstream injection orifice (12B) situated downstream from the balloon (5) and/or at least one upstream injection orifice (12B) situated upstream from the balloon (5); and
- a third leaktight internal conduit (8) opening out, on the one hand, at the proximal end and on the other hand into the one or more injection orifice(s) (12) in such a way as to permit the injection of a contrast agent through the one or more injection orifice(s) (12) when the guide wire is in the first conduit and the inflatable balloon (5) is deflated, with the third conduit (8) not opening on the first conduit (6).

2. An angioplasty device according to claim 1, **characterized in that** the injection orifices (12) are distributed radially about the tubular body (1).

3. An angioplasty device according to any one of the preceding claims, **characterized in that** the one or more injection orifice (s) (12) include at least one downstream injection orifice (12B) situated downstream from the balloon (5).

4. An angioplasty device according to any one of the preceding claims, **characterized in that** the one or more injection orifice (s) (12) include at least one upstream injection orifice (12B) situated upstream from the balloon (5).

5. An angioplasty device according to any one of the preceding claims, **characterized in that** the first, second and third conduits (6, 7, 8) are concentric.

6. An angioplasty device according to any one of the preceding claims, **characterized in that** the first, second and third conduits (6, 7, 8) are arranged side by side.

7. An angioplasty device according to any one of the preceding claims, **characterized in that** the first conduit (6) is of a circular cross section and has a diameter equal to that of the guide wire.

8. An angioplasty device according to any one of the preceding claims, **characterized in that** the proximal end (3) of the device is provided with three connector pieces (9, 10, 11), with each connector piece (9, 10, 11) being attached in a leaktight manner to one of the conduits (6, 7, 8).

9. An angioplasty device according to any one of the preceding claims, **characterized in that** the tubular body (1) comprises a fourth conduit, for instance to inject drugs or active agents into blood.

10. An angioplasty device according to any one of the preceding claims, **characterized in that** the first conduit opens out, on the one hand, at the proximal end and on the other hand, at the distal end.

11. An angioplasty device according to any one of claims 1 to 10, **characterized in that** the first conduit opens out at an intermediate position between the proximal end and the inflatable balloon (5) and on the other hand, at the distal end.
